(19) Europäisches Patentamt / European Patent Office / Office européen des brevets

(11) **EP 2 655 302 B1**

(12) **FASCICULE DE BREVET EUROPEEN**

(45) Date de publication et mention de la délivrance du brevet:
**21.10.2015 Bulletin 2015/43**

(21) Numéro de dépôt: **11808902.8**

(22) Date de dépôt: **16.12.2011**

(51) Int Cl.:
*C07C 29/132* *(2006.01)*   *C07C 45/60* *(2006.01)*
*C07C 31/20* *(2006.01)*   *C07C 49/17* *(2006.01)*

(86) Numéro de dépôt international:
**PCT/FR2011/000662**

(87) Numéro de publication internationale:
**WO 2012/085362 (28.06.2012 Gazette 2012/26)**

(54) **PROCÉDÉ DE TRANSFORMATION DE BIOMASSE LIGNOCELLULOSIQUE OU DE CELLULOSE PAR DES CATALYSEURS À BASE D'OXYDE D'ÉTAIN ET/OU D'OXYDE D'ANTIMOINE ET D'UN MÉTAL CHOISI DANS LES GROUPES 8 À 11.**

VERFAHREN ZUR UMWANDLUNG VON CELLULOSE ODER LIGNOCELLULOSE MIT KATALYSATOREN AUF DER BASIS VON ZINNOXID UND/ODER ANTIMONOXYD UND EINES METALLS AUS GRUPPE 8 BIS 11

PROCESS FOR CONVERTING CELLULOSE OR LIGNOCELLULOSIC BIOMASS USING CATALYSTS BASED ON TIN OXIDE AND/OR ON ANTIMONY OXIDE AND ON A METAL SELECTED FROM GROUPS 8 TO 11

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **22.12.2010 FR 1005023**

(43) Date de publication de la demande:
**30.10.2013 Bulletin 2013/44**

(73) Titulaires:
• **IFP Énergies nouvelles**
  **92852 Rueil-Malmaison Cedex (FR)**
• **CNRS**
  **75794 Paris Cedex 16 (FR)**

(72) Inventeurs:
• **CHAMBON, Flora**
  **F-69500 Bron (FR)**
• **ESSAYEM, Nadine**
  **F-38540 Saint-Just Chaleyssin (FR)**
• **RATABOUL, Franck**
  **F-69008 Lyon (FR)**
• **PINEL, Catherine**
  **F-69007 Lyon (FR)**
• **CABIAC, Amandine**
  **F-69007 Lyon (FR)**
• **GUILLON, Emmanuelle**
  **F-69390 Vourles (FR)**

(56) Documents cités:
**WO-A2-2010/101637    US-A- 2 488 722**
**US-A- 5 354 914**

• **DENG TIAN YIN AND CO: "Cellulose conversion to polyols on supported Ru catalysts in aqueous basic solution", SCIENCE CHINA, CHEMISTRY, vol. 53, no. 7, 4 mai 2010 (2010-05-04), pages 1476-1480, XP002655640,**
• **REGINA PALKOVITS ET AL: "Hydrogenolysis of cellulose combining mineral acids and hydrogenationcatalysts", GREEN CHEMISTRY, ROYAL SOCIETY OF CHEMISTRY, CAMBRIDGE, GB, vol. 12, no. 6, 1 janvier 2010 (2010-01-01), pages 972-978, XP009150924, ISSN: 1463-9262, DOI: DOI:10.1039/C000075B [extrait le 2010-05-21]**
• **SHIMIZU K ET AL: "Effects of Bronsted and Lewis acidities on activity and selectivity of heteropolyacid-based catalysts for hydrolysis of cellobiose and cellulose", GREEN CHEMISTRY, ROYAL SOCIETY OF CHEMISTRY, CAMBRIDGE, GB, vol. 11, no. 10, 1 janvier 2009 (2009-01-01), pages 1627-1632, XP009150892, ISSN: 1463-9262, DOI: DOI:10.1039/B913737H [extrait le 2009-07-24]**

• **TUSHAR P VISPUTE ET AL: "Production of hydrogen, alkanes and polyols by aqueous phase processing of wood-derived pyrolysis oils", GREEN CHEMISTRY, ROYAL SOCIETY OF CHEMISTRY, CAMBRIDGE, GB, vol. 11, no. 9, 1 janvier 2009 (2009-01-01), pages 1433-1445, XP009150923, ISSN: 1463-9262**

Remarques:
  Le dossier contient des informations techniques présentées postérieurement au dépôt de la demande et ne figurant pas dans le présent fascicule.

**Description**

**DOMAINE DE L'INVENTION**

L'invention concerne un procédé de transformation de biomasse lignocellulosique ou de cellulose directement en produits valorisables que sont l' hydroxyacétone et le propylène glycol mettant en oeuvre des catalyseurs hétérogènes.

**ART ANTERIEUR**

Depuis quelques d'années, il existe un vif regain d'intérêt pour l'incorporation de produits d'origine renouvelable au sein des filières carburant et chimie, en complément ou en substitution des produits d'origine fossile. Une voie possible est la conversion de la cellulose contenue dans la biomasse lignocellulosique en produits ou intermédiaires chimiques, et en particulier en hydroxyacétone et en propylène glycol.

Le terme biomasse lignocellulosique (BLC) ou lignocellulose englobe plusieurs produits présents en quantités variables selon son origine : la cellulose, l'hémicellulose et la lignine. L'hémicellulose et la cellulose constituent la partie carbohydrate de la lignocellulose. Ce sont des polymères de sucres (pentoses et hexoses). La lignine est une macromolécule riche en motifs phénoliques. Par biomasse lignocellulosique, on entend par exemple les produits issus de l'exploitation forestière et les sous-produits issus de l'agriculture comme la paille ainsi que certains végétaux dédiés à haut rendement agricole.

La production de produits chimiques à partir de biomasse lignocellulosique permet à la fois de réduire la dépendance énergétique vis-à-vis du pétrole et de préserver l'environnement à travers la diminution des émissions de gaz à effet de serre sans utiliser de ressources destinées aux usages alimentaires.

La transformation directe de biomasse lignocellulosique ou de cellulose en produits ou intermédiaires chimiques, et en particulier en hydroxyacétone et en propylène glycol est une voie particulièrement intéressante. Par transformation directe, on entend la transformation en une étape de biomasse lignocellulosique ou de la cellulose, éventuellement prétraitée, vers des produits valorisables et en particulier en hydroxyacétone et en propylène glycol.

L'hydroxyacétone, ou acétol a pour formule chimique $C_3H_6O_2$ et sa structure se reflète dans son nom systématique, la 1-hydroxy-propanone. L'hydroxyacétone est utilisé par exemple comme intermédiaire chimique et comme monomère pour la synthèse des polyols, mais aussi comme solvant chimique.

La production de l'hydroxyacétone peut se faire par voie chimique ou par voie biologique. Les voies chimiques de production de l'hydroxyacétone connues de l'homme du métier se font via la transformation d'intermédiaires pétrochimiques telle que l'hydratation du propylène. L'oxydation du 1,2-propanediol produit par voie biologique peut également conduire à la formation de l'hydroxyacétone.

Le propylène glycol, ou propan-1,2-diol a pour formule chimique $C_3H_8O_2$ et sa structure se reflète dans son nom systématique, le 1,2-dihydroxypropane. Les applications du propylène glycol sont nombreuses et diverses : on citera par exemple son utilisation en tant qu'additif alimentaire, émulsifiant, intermédiaire de polyesters insaturés, mais aussi celle de liquide de refroidissement ou son utilisation dans l'industrie textile.

La production de propylène glycol est industriellement mise en oeuvre par hydratation de l'oxyde de propylène.

La valorisation de la biomasse lignocellulosique ou de la cellulose contenue dans la biomasse par catalyse hétérogène est décrite dans la littérature. La demande de brevet EP-A1-B 2011 569 décrit l'hydrolyse de la cellulose en sorbitol et en mannitol, en milieu aqueux avec des catalyseurs métalliques hétérogènes.

La demande de brevet WO 03/035582 décrit l'hydrogénolyse du sorbitol à 200°C en utilisant des catalyseurs (Ni, Re)/C qui conduit à des rendements de 30% en diols comme l'éthylène glycol et le propylène glycol.

L'obtention de propylène glycol par traitement de la cellulose en conditions hydrothermales en présence de catalyseurs hétérogènes Ru/C est observée par Luo et al. (Angew. Chem. Int. Ed. 2007, 46, 7636-7639). Le rendement carbone maximal obtenu en propylène glycol est de 2,2% poids. pour des réactions menées à une température de 245°C, une pression en $H_2$ de 6 MPa, en milieu aqueux. Dans ces conditions de réaction, la conversion de la cellulose est de 39% environ. La production d'hydroxyacétone n'est pas reportée.

Ji et al. (Angew. Chem. Int. Ed. 2008, 47, 8510-8513) ont également étudié la réaction de transformation de la cellulose en milieu hydrothermal en utilisant des catalyseurs à base de carbures de tungstène supporté sur charbon avec du nickel comme promoteur, mettant en avant une bonne sélectivité en éthylène glycol et en sorbitol avec ce type de catalyseur. Les conditions opératoires sont une température de l'ordre de 245°C, une pression en hydrogène de 6MPa, en présence d'eau. Le rendement massique maximal en 1,2-propylène glycol est de 7,7% pour un catalyseur nickel carbure de tungstène supporté sur charbon. En utilisant un catalyseur de type 2,5% $Pt/Al_2O_3$, le rendement massique en propylène glycol est de 9,3%. La conversion de la cellulose est totale dans les deux cas.

Là encore, la production d'hydroxyacétone n'est pas mentionnée.

De même, Zhang et al. (Chem. Commun., 2010, 46, 862-864) ont récemment mis en avant la conversion directe de la cellulose en éthylène glycol par des catalyseurs à base de carbure de tungstène supporté sur des matériaux carbonés de silice commerciale. Au cours de ces expériences, avec une température de l'ordre de 245°C, une pression en hydrogène de 6 MPa, en milieu aqueux, le rendement massique maximal en propylène glycol est de 8,4%, la conversion de la cellulose est totale et la formation d'hydroxyacétone n'est pas observée.

La même équipe de recherche a mis en avant la conversion de la cellulose en éthylène glycol par des catalyseurs nickel tungstène supporté sur une silice mésoporeuse de type SBA-15 (Zheng et al. ChemSusChem., 2010, 3, 63-66). Une fois encore, une conversion totale de la cellulose est obtenue avec un rendement massique maximal en propylène glycol faible (de l'ordre de 4%) et sans formation d'hydroxyacétone.

Le brevet US 2 488 722 décrit un procédé de transformation de cellulose en hydroxyacétone et en propylène glycol dans lequel la cellulose est dissoute dans le réactif de Schweizer c'est à dire dans une solution ammoniacale d'oxyde de cuivre, puis la solution est réduite former un mélange d'alcool isopropylique, de propylène glycol et d'hydroxyacétone.

Le document Science china chemistry, 2010, 53, 1476-4780 décrit un procédé de transformation de cellulose en polyols dans lequel la cellulose est hydrogénolysée à haute température et haute pression dans un milieu aqueux basique en présence de ruthénuim pour conduire à un mélange d'éthylène glycol, de propylène glycol, de 1,2,5-pentanetriol et d'acétol.

Enfin, des procédés de conversions thermiques et non catalytiques comme la pyrolyse ou la liquéfaction directe de la lignocellulose conduisent à la production de liquéfiats de biomasse. La présence minoritaire d'hydroxyacétone est quelquefois notée. On citera par exemple Patwardhan et al. Bioresource Technology, 2010, 101, 4646-4655. Néanmoins, ces procédés non catalytiques sont de part leurs conditions d'application (température, pression) très éloignés du procédé faisant l'objet de l'invention.

Il n'est donc pas rapporté dans la littérature de procédé qui permet une transformation directe de la cellulose ou plus largement de la biomasse lignocellulosique, éventuellement prétraitée, en produits valorisables que sont l'hydroxyacétone et le propylène glycol à haute sélectivité au moyen de catalyseurs hétérogènes du type de ceux décrits dans la présente invention.

## RESUME DE L'INVENTION

Les demandeurs ont découvert un procédé de transformation directe de la cellulose, présente dans la biomasse lignocellulosique, éventuellement prétraitée, en hydroxyacétone et en propylène glycol, mettant en oeuvre un catalyseur hétérogène à base d'oxyde d'étain et/ou d'oxyde d'antimoine, dispersé sur un support, et contenant au moins un élément à l'état métallique choisi parmi Pt, Ni, Ru et Cu.

## DESCRIPTION DETAILLEE DE L'INVENTION

L'invention concerne un procédé de transformation de la biomasse lignocellulosique ou de cellulose en hydroxyacétone et en propylène glycol, dans lequel la biomasse lignocellulosique ou la cellulose est mise en contact, en conditions hydrothermales, sous atmosphère réductrice, avec un catalyseur hétérogène à base d'oxyde d'étain et/ou d'oxyde d'antimoine et contenant au moins un élément à l'état métallique choisi parmi Pt, Ni, Ru, Cu, ledit catalyseur présentant des sites de type Lewis, ladite teneur en sites acides de Lewis étant supérieure à 50% de la teneur en sites acides totale, ledit catalyseur étant dispersé sur un support à base d'oxydes choisi parmi les oxydes d'aluminium, et/ou de zirconium, et/ou de titane, et/ou de niobium et/ou de silicium, ledit procédé étant effectué à une température comprise entre 160 et 250°C, et à une pression comprise entre 0,5 et 20 Mpa.

Le procédé selon l'invention permet d'obtenir de façon sélective un mélange de produits comprenant de l'hydroxyacétone et du propylène glycol, en quantité importante.

Lors de la transformation de la charge, il est possible d'obtenir un mélange de différents produits comprenant notamment du glucose, du sorbitol, de l'acide lactique, de l'acide formique, de l'acide lévulinique, de l'acide acétique, de l'hydroxyacétone, du propylène glycol, du 2,5-hexanedione, du 5-HMF (hydroxyméthylfurfural), de 1,2-hexanediol, des produits solubles et des oligosaccharides et polymères solubles.

Le procédé permet d'obtenir des conversions élevées du réactif et des sélectivités importantes, en particulier des rendements élevés en hydroxyacétone et en propylène glycol, tout en limitant la formation d'oligosaccharides ou de polymères hydrosolubles. Ces conversions et sélectivités ne sont obtenues qu'en conditions hydrothermales (présence d'eau), qu'en opérant sous atmosphère réductrice, et qu'en présence de catalyseurs à base d'oxydes d'étain et/ou d'oxyde d'antimoine présentant des propriétés acides de type Lewis et contenant un élément à l'état métallique choisi parmi Pt, Ni, Ru, Cu. En effet, les catalyseurs solides ayant majoritairement une acidité de Brønsted favorisent la production d'oligosaccharides solubles et/ou polymères solubles, présentant une moindre sélectivité en intermédiaires chimiques désirés. D'autre part, les catalyseurs à base d'oxyde d'étain et/ou d'oxyde d'antimoine présentant des propriétés acides de type Lewis et ne contenant pas de métal ne conduisent pas à la formation des intermédiaires chimiques désirés, mais permettent d'obtenir sélectivement de l'acide lactique.

Ainsi, le rendement molaire en hydroxyacétone et en propylène glycol est supérieur au rendement de chacun des autres produits obtenus lors de la transformation de la biomasse lignocellulosique, et est également supérieur à la somme des rendements des différents produits pris dans leur ensemble.

Ledit support est de préférence à base d'oxyde(s) choisis parmi les oxydes d'aluminium et/ou de zirconium et/ou de titane et/ou de niobium et/ou de silicium.

La teneur en sites acides de type Lewis du catalyseur est supérieure à 50 % de la teneur en sites acides totaux. Sous le terme teneur totale en sites acides, on comprend la somme des sites acides de Lewis et des sites acides de Bronsted.

L'utilisation de ces catalyseurs permet d'obtenir directement des produits valorisables, que sont l'hydroxacétone et le propylène glycol, en haute sélectivité tout en limitant la production d'oligosaccharides et polymères solubles.

Le procédé selon la présente invention permet d'améliorer également la conversion de la cellulose présente dans la biomasse lignocellulosique.

**La charge**

La biomasse lignocellulosique est essentiellement constituée de trois constituants naturels présents en quantités variables selon son origine : la cellulose, l'hémicellulose et la lignine.

La cellulose $(C_6H_{10}O_5)_n$ représente la majeure partie (40-60%) de la composition de la biomasse lignocellulosique. C'est un homopolymère linéaire semi-cristallin du glucose. La cellulose est insoluble dans l'eau à température et pression ambiantes.

L'hémicellulose est le deuxième carbohydrate en quantité après la cellulose et constitue 20 à 40% en poids de la biomasse lignocellulosique. Contrairement à la cellulose, ce polymère est constitué en majorité de monomères de pentoses (cycles à cinq atomes) et hexoses (cycles à 6 atomes). L'hémicellulose est un hétéropolymère amorphe avec un degré de polymérisation inférieur à celui de la cellulose (30-100)

La lignine est une macromolécule amorphe présente dans les composés lignocellulosiques dans des proportions variables selon l'origine du matériau (paille ~ 15%, bois : 20-26%). Sa fonction est le renforcement mécanique, l'hydrophobisation et le soutien des végétaux. Cette macromolécule riche en motifs phénoliques peut être décrite comme résultant de la combinaison de trois unités monomères de type propyl-méthoxy-phénols. Sa masse molaire varie de 5000 g/mol à 10000 g/mol pour les bois durs et atteint 20000 g/mol pour les bois tendres.

La matière première lignocellulosique peut être constituée de bois ou de déchets végétaux. D'autres exemples de matière biomasse lignocellulosique sont les résidus d'exploitation agricole (paille, herbes, tiges, noyaux, coquilles...), les résidus d'exploitation forestière (produits de première éclaircie, écorces, sciures, copeaux, chutes...), les produits d'exploitation forestière, les cultures dédiées (taillis à courte rotation), les résidus de l'industrie agro-alimentaire (résidu de l'industrie du cotton, bambou, sisal, banane, maïs, panicum virgatum, alfalfa, noix de coco, bagasse...), les déchets organiques ménagers, les déchets des installations de transformation du bois, les bois usagés de construction, du papier, recyclé ou non.

La charge utilisée dans le procédé selon l'invention est de la biomasse lignocellulosique ou de la cellulose. La cellulose utilisée peut être cristalline, partiellement amorphe ou amorphe.

La charge biomasse lignocellulosique peut être utilisée sous sa forme brute, c'est-à-dire dans son intégralité de ses trois constituants cellulose, hémicellulose et lignine. La biomasse brute se présente généralement sous forme de résidus fibreux ou poudre. En général, elle est broyée ou déchiquetée pour permettre son transport.

La charge biomasse lignocellulosique peut aussi être utilisée sous sa forme prétraitée, c'est-à-dire sous une forme contenant au moins une partie cellulosique après extraction de la lignine et/ou de l'hémicellulose.

La biomasse subit de préférence un prétraitement afin d'augmenter la réactivité et l'accessibilité de la cellulose au sein de la biomasse avant sa transformation. Ces prétraitements sont de nature mécanique, thermochimique, thermo-mécanico-chimique et/ou biochimique et provoquent la decristallinisation partielle ou totale de la cellulose, la solubilisation totale ou en partie de l'hémicellulose et/ou de la lignine ou l'hydrolyse partielle de l'hémicellulose suivant le traitement.

La charge biomasse lignocellulosique peut également être prétraitée afin d'être sous la forme d'oligomères hydrosolubles. Ces prétraitements sont de nature mécanique, thermochimique, thermo-mécanico-chimique et/ou biochimique. Ils provoquent la décristallisation et la solubilisation de la cellulose sous forme d'oligomères hydrosolubles.

Les traitements mécaniques vont au delà du simple déchiquetage car ils modifient la structure chimique des constituants. Ils améliorent l'accessibilité et la réactivité de la cellulose par sa décristallisation partielle ou totale et par l'augmentation de la surface d'échange. Les traitements mécaniques incluent la réduction de la taille des fibres ou particules élémentaires, par exemple par mise en copeaux de la biomasse à l'aide d'une coupeuse, par broyage de la biomasse (ajustement de la granulométrie), déstructuration des copeaux sur presse ou défibrage par abrasion des copeaux, après préchauffage. Les traitements mécaniques peuvent être opérés en mode décentralisé près de la production de la biomasse ou en mode centralisé alimentant directement la transformation.

Les traitements thermochimiques incluent la cuisson de la biomasse à haute température (150-170°C) en milieu acide dilué (principalement acide sulfurique, mais aussi acide phosphorique, acide acétique ou acide formique), en milieu alcalin (soude, sulfites, chaux...) ou en milieu oxydant (oxydation humide à l'air ou à l'oxygène ; peroxyde en milieu alcalin ; acide peracétique). Les autres traitements thermochimiques incluent des traitements aux solvants (éthanol à chaud) ou la torréfaction qui peut se définir comme une pyrolyse à température modérée et temps de séjour contrôlé car elle s'accompagne d'une destruction partielle de la matière lignocellulosique. Les technologies connues pour la torréfaction sont par exemple le four tournant, le lit mobile, le lit fluidisé, la vis sans fin chauffée, le contact avec de billes métalliques apportant la chaleur. Ces technologies peuvent éventuellement utiliser un gaz circulant à co ou contre courant comme de l'azote ou tout autre gaz inerte dans les conditions de la réaction.

Les traitements thermo-mécanico-chimiques incluent les traitements à la vapeur (explosion à la vapeur appelés encore hydrolyse flash ou "steam-explosion"), le traitement AFEX (ammonia fiber explosion) à l'ammoniaque ou l'extrusion bi-vis avec des réactifs chimiques divers.

Le prétraitement permet de préparer la biomasse lignocellulosique en séparant la partie carbohydrate de la lignine et ajustant la taille des particules de biomasse à traiter. La taille des particules de biomasse après le prétraitement est généralement inférieure à 5 mm, de préférence inférieure à 500 microns.

## Le catalyseur

Les catalyseurs utilisés pour la transformation de la biomasse lignocellulosique ou de la cellulose selon la présente invention sont à base d'oxyde d'étain et/ou d'oxyde d'antimoine, dispersés à la surface d'un support, et contiennent au moins un élément à l'état métallique choisi r parmi Pt, Ni, Ru, Cu.

D'une manière générale, l'acidité d'un catalyseur est la résultante de deux types d'acidité combinés : une acidité de Lewis, caractérisée par la présence d'une lacune électronique sur un atome, et une acidité de Brønsted, caractérisée par une aptitude à céder un proton. La nature des sites acides peut se caractériser par adsorption de pyridine suivie par spectroscopie IR conformément à la méthode décrite dans [M. Guisnet, P. Ayrault, C. Coutanceau, M.F. Alvarez, J. Datka, J. Chem. Soc., Faraday Trans. 93, 1661 (1997)].

Les solides utilisés dans le procédé selon l'invention sont caractérisés par des propriétés acides superficielles majoritairement de type Lewis.

Le catalyseur a une teneur en sites acides de Lewis supérieure à 50 % de l'acidité totale dudit solide. Les sites acides de type Lewis sont associés à la présence d'espèces étain et/ou antimoine coordinativement insaturées mais aussi aux espèces caractéristiques du support, comme par exemple $Al^{3+}$, $Zr^{4+}$, $Ti^{4+}$, $Nb^{5+}$. L'acidité de Lewis peut être caractérisée par exemple par spectroscopie IR.

Le support utilisé pour disperser de l'étain et/ou de l'antimoine et un élément particulier à l'état métallique est un oxyde, choisi de préférence parmi l'alumine, la zircone, l'oxyde de niobium, l'oxyde de titane, la silice, les zéotypes comme par exemple les zéolithes, les aluminophosphates, les composés mésostructurés seuls ou en mélange préparés selon toute technique connue de l'Homme du métier. Par exemple, les supports peuvent être synthétisés par précipitation, synthèse sol-gel suivi d'un traitement thermique. Les solides obtenus présentent l'avantage d'être stables thermiquement et en conditions hydrothermales.

La teneur en étain et/ou en antimoine est comprise entre 1 à 100% en poids, de préférence entre 1 et 50%, de préférence entre 1 et 30%, et encore plus préférentiellement entre 1 et 20% poids, les pourcentages étant exprimés en % poids de métal par rapport à la masse totale du catalyseur.

Les précurseurs d'étain ou d'antimoine sont choisis parmi les hydrures, les halogénures (les chlorures, les bromures, les iodures, les fluorures), les oxydes, les sulfures ou les composés organométalliques respectivement d'étain ou d'antimoine. Les chlorures d'étain sont les précurseur usuels. L'utilisation de chlorure d'étain en solution dans de l'acide chlorhydrique est préférée. Parmi les composés organométalliques d'étain, on citera les alkyl étain et plus particulièrement le tétrabutylétain.

La préparation de catalyseurs à base d'oxyde d'étain et/ou d'antimoine peut être réalisée selon toute technique connue de l'Homme du métier, comme par exemple l'échange ionique, l'imprégnation à sec ou l'imprégnation en excès. Une méthode de préparation consiste en une imprégnation d'une solution d'acide stannique et/ou d'oxyde d'antimoine et du support, de préférence à base d'oxyde, suivie d'une calcination.

La présence d'étain et/ou d'antimoine sur le support entraîne la formation d'oxyde d'étain et/ou d'antimoine.

L'élément à l'état métallique présent dans le catalyseur mis en oeuvre selon la présente invention est un métal choisi parmi Pt, Ni, Ru, Cu. De manière plus préférée, l'élément est choisi parmi le Pt, Ni et Ru. De manière très préférée, il s'agit du platine.

Les précurseurs de métal peuvent être, sans en limiter l'origine, des complexes organiques métalliques, des sels de métaux. On citera par exemple pour les sels de métaux, les chlorures métalliques, les nitrates métalliques.

L'introduction du métal peut être réalisée par toute technique connue de l'Homme du métier comme par exemple l'échange ionique, l'imprégnation à sec, l'imprégnation par excès, le dépôt en phase vapeur, etc. L'introduction de métal peut être réalisée avant ou après la mise en forme du catalyseur à base d'oxyde d'étain et/ou d'oxyde d'antimoine

dispersé sur un support.

La teneur pondérale de l'élément métallique introduit est avantageusement comprise entre 0,01 et 10% poids, et préférentiellement entre 0,05 et 5% poids par rapport à la masse totale du catalyseur.

L'étape d'introduction de l'élément métallique est suivie d'une étape de traitement thermique. Le traitement thermique est avantageusement réalisé entre 300°C et 700°C. L'étape de traitement thermique peut être suivie d'un traitement de réduction en température. Le traitement thermique réducteur est avantageusement réalisé à une température comprise entre 200°C et 600°C sous flux ou atmosphère d'hydrogène. L'étape de réduction peut être réalisée in-situ c'est-à-dire dans le réacteur où se déroule la réaction, avant l'introduction de la charge réactionnelle. La réduction peut également être réalisée ex-situ.

Les catalyseurs utilisés dans la présente invention peuvent être sous forme de poudre, d'extrudés, de billes ou de pastilles. La mise en forme peut être réalisée avant ou après l'introduction du métal et/ou de l'étain et/ou de l'antimoine.

A l'issue des traitements thermiques et/ou réducteurs, la présence d'alliage formé entre l'oxyde d'étain et/ou l'oxyde antimoine et l'élément à l'état métallique choisi dans les groupes 8 à 11 de la classification périodique peut être observée. Par exemple, la présence d'alliage platine étain et/ou platine/antimoine peut être observée.

Les catalyseurs utilisés dans la présente invention sont caractérisés par les techniques connues de l'homme du métier.

Le catalyseur utilisé selon l'invention est stable et régénérable, c'est-à-dire qu'il ne subit pas de lixiviation pendant la réaction. A l'issue d'une étape de lavage ou de combustion des espèces hydrocarbonées déposées sur le catalyseur après réaction, le catalyseur présente les mêmes performances catalytiques initiales.

**Procédé de transformation**

Le procédé de transformation de la biomasse lignocellulosique ou de la cellulose selon l'invention comprend la réaction dans un milieu contenant de l'eau en présence de la composition catalytique conforme à l'invention.

Par milieu contenant de l'eau, on désigne les milieux liquides conventionnels comme les alcools tels que par exemple le méthanol ou l'éthanol, et l'eau et les milieux non conventionnels comme les liquides ioniques ou les milieux supercritiques de densité type liquide.

La teneur massique en eau dans le milieu est généralement supérieure à 1%. De préférence, le milieu est de l'eau.

Le procédé de transformation de la biomasse lignocellulosique ou de la cellulose selon l'invention est réalisé sous atmosphère réductrice, de préférence sous atmosphère d'hydrogène. L'hydrogène peut être utilisé pur ou en mélange.

Le procédé est opéré à des températures comprises entre 160°C et 250°C, de préférence entre 175°C et 250°C, et à une pression comprise entre 0,5 MPa et 20 MPa, de préférence entre 2 MPa et 10 MPa.

Généralement la réaction peut être opérée selon différents modes de réalisation. Ainsi, la réaction peut être mise en oeuvre en discontinu ou en continu, par exemple en lit fixe. On peut opérer en réacteur fermé ou en réacteur semi-ouvert.

Le catalyseur est introduit dans le réacteur à raison d'une quantité correspondant à un rapport massique biomasse/catalyseur compris entre 1 et 1000, de préférence entre 1 et 500, de préférence entre 1 et 100, de préférence entre 1 et 50 et encore préférentiellement entre 1 et 25.

Le catalyseur introduit dans le réacteur peut subir une étape de traitement thermique réducteur avant l'introduction de la charge réactionnelle. Le traitement thermique réducteur est réalisé à une température comprise entre 200°C et 600°C sous flux ou atmosphère d'hydrogène.

La biomasse est introduite dans le procédé à raison d'une quantité correspondant à un rapport massique (milieu contenant de l'eau)/biomasse compris entre 1 et 1000, de préférence entre 1 et 500 et encore préférentiellement entre 5 et 100. Le taux de dilution de la biomasse est dont entre 1:1 et 1:1000, de préférence entre 1:1 et 1:500 et encore préférentiellement entre 1:5 et 1:100.

Si l'on choisit un procédé en continu, la vitesse massique horaire (débit de charge massique/masse de catalyseur) est entre 0,01 et 5 $h^{-1}$, de préférence entre 0,02 et 2 $h^{-1}$.

**Les produits obtenus et leur mode d'analyse**

Après la réaction, le milieu réactionnel est prélevé et centrifugé. Le liquide réactionnel est ensuite analysé par chromatographie liquide sous haute pression (HPLC) en utilisant la réfractométrie pour déterminer la teneur en produits de conversion de la solution aqueuse.

Les produits de la réaction sont solubles dans l'eau. Ils sont constitués de monosaccharides et de leurs dérivés, d'oligosaccharides, mais aussi de polymères solubles formés par combinaisons successives des dérivés des monosaccharides.

Par monosaccharides, on désigne les sucres simples (hexoses, pentoses) produits par dépolymérisation complète de la cellulose et/ou hémicellulose, en particulier, le glucose, le mannose, le xylose, le fructose....

Par dérivés des monosaccharides on désigne les produits pouvant être obtenus par déshydratation, isomérisation, réduction ou oxydation :

- des sucres alcools, des alcools et polyols : en particulier le sorbitol, le xylitol, le glycérol, l'éthylène glycol, le propylène glycol, l'éthanol, l'hydroxyacétone...
- des cétones, des hexane-diones : la 2,5-hexanedione, l'hydroxyacétone...
- des acides carboxyliques et leurs esters, des lactones : l'acide formique, acide lévulinique, lévulinates d'alkyles, acide lactique, lactates d'alkyles, acide glutarique, glutarates d'alkyles, l'acide 3-hydroxypropanoique, la 3-hydroxybutyrolactone, la γ-butyrolactone,
- des éthers cycliques : par exemple le tétrahydrofurane (THF), le méthyltétrahydrofurane (Me-THF), le furane dicarboxylique acide, le 5-(hydroxyméthyl)furfural...

Par oligosaccharide, on désigne un carbohydrate ayant pour composition $(C_6H_{10}O_5)_n$ où n est supérieur à 1, obtenu par hydrolyse partielle de la cellulose, ou de l'hémicellulose, ou de l'amidon.

Par polymères solubles, on désigne tous les produits issus de la condensation entre monosaccharides, oligosaccharides et/ou dérivés des monosaccharides.

La quantité des produits de réaction solubles dans l'eau (monosaccharides et dérivés, oligosaccharides, polymères solubles) est déterminée par l'analyse COT (Carbone Organique Total) qui consiste en la mesure du carbone en solution. La quantité des monosaccharides et leurs dérivés est déterminée par analyses HPLC.

La conversion est définie comme le pourcentage de solubilisation de la biomasse ou de la cellulose et est calculée selon l'équation suivante :

$$C = 100 * C_{solubilisé} / C_{initial}$$

dans laquelle $C_{solubilisé}$ représente la quantité de carbone solubilisé analysée par COT (mg) et $C_{initial}$ la quantité de carbone en début de réaction contenue dans la biomasse ou cellulose solide.

Les rendements molaires en dérivés du glucose sont calculés au moyen de l'analyse HPLC. Chaque composé est corrigé du nombre d'atome de carbone contenu dans l'unité glucose.

Les rendements molaires en un dérivé i sont calculés comme suit :

$$Rdti = 100* (nC_{Pi}/6)*( P_i/Glu_0)$$

où $nC_{pi}$ représente le nombre d'atome de carbones du dérivé i, Pi le nombre de moles du produit $P_i$ et $Glu_0$ le nombre de moles d'unités glucose contenues dans la biomasse ou la cellulose en début de réaction.

La formation d'oligosaccharides et de polymères solubles correspondent à une perte de carbone. Cette perte de carbone est déduite des analyses COT et HPLC. Le rendement en oligosaccharides et polymères solubles est calculé selon l'équation suivante :

$$Rdt_{olig} = C - \sum rdt_i$$

où C représente la conversion de la cellulose et $\sum rdt_i$ la somme des rendements molaires de tous les monosaccharides et leurs dérivés analysés par HPLC.

**EXEMPLES**

Exemple 1 : Préparation du catalyseur C1 à base d'oxyde d'étain dispersé sur un support à base d'oxyde d'alumine (non conforme à l'invention): .

Le catalyseur est préparé en utilisant comme matière première de l'hydroxyde d'aluminium et du chlorure d'étain pentahydraté. 5,0 g d'hydroxyde d'aluminium sont soumis à une imprégnation à humidité naissante avec une solution aqueuse de chlorure d'étain (3,0g de $SnCl_4$, $5H_2O$ dans 4,5g d'eau). Le solide obtenu est ensuite séché à 80°C pendant 24 heures.

Ensuite, le solide est calciné sous débit d'air sec à la température de 700°C pendant 3 heures. A l'issue de ces traitements, le catalyseur C1 contient 15% poids d'étain. La proportion de sites acides de Lewis du catalyseurs C1 est supérieure à 90%

Exemple 2 : Préparation du catalyseur C2 à base d'oxyde d'étain dispersé sur un support à base d'oxyde d'alumine et de platine (conforme à l'invention

1 g de catalyseur C1 est traité sous vide 1 h à 100°C. Une solution aqueuse d'acide hexachloroplatinique $H_2PtCl_6.xH_2O$ à 8% pds (1,3mL, 0,525g) est ensuite ajoutée au catalyseur C1. Le mélange est agité pendant une heure. La solution aqueuse est évaporée. Le solide obtenu est en premier lieu séché à l'étuve à 110°C pendant 24h puis traité thermiquement sous débit d'azote sec à la température de 550°C pendant deux heures puis réduit sous flux d'hydrogène à 300°C pendant deux heures.

Le catalyseur C2 ainsi obtenu contient 2,2% poids de platine et 15% poids d'étain. La proportion de sites acides de Lewis du catalyseurs C2 est supérieure à 90%.

Exemple 3 : préparation d'un catalyseur C3 à base de platine supporté sur silice (non conforme à l'invention) :

La matière première utilisée est le support commercial $SiO_2$ Alfa Aesar. Typiquement, une solution aqueuse de platine tétramine (1,3mL; 0,171g) est ajoutée à température ambiante à la silice (1g) préalablement désorbée sous vide (1 h, 100°C). Le mélange est agité pendant une heure puis est ensuite évaporé. Le solide obtenu est ensuite mis à sécher à l'étuve à 110°C pendant 24h. Ensuite le catalyseur est calciné sous débit d'azote sec à la température de 500°C pendant deux heures puis réduit sous flux d'hydrogène à 300°C pendant deux heures.

Le catalyseur C3 obtenu contient 1,6% poids de platine.

Exemple 4 : Transformation de la cellulose mettant en oeuvre les catalyseurs préparés dans les exemples 1, 2 et 3.

Cet exemple concerne la conversion de la cellulose à partir des catalyseurs C1, C2 et C3 pour la production de produits C3 valorisables, et, que sont l'hydroxyacétone et le propylène glycol.

Dans un autoclave de 100mL, on introduit 65mL d'eau, 1,6g de cellulose Avicel® (70% cristallinité) et 0,68g de catalyseur C1, C2 ou C3. L'autoclave est chauffé à 190°C et une pression de 5 MPa d'hydrogène est introduite. Après 24h de réaction, le milieu réactionnel est prélevé et centrifugé. Le liquide réactionnel est ensuite analysé par chromatographie liquide sous haute pression (HPLC) en utilisant la réfractométrie pour déterminer la teneur en produits de conversion de la solution aqueuse.

La conversion de la cellulose est également réalisée en absence de catalyseur, à titre comparatif.

Les résultats obtenus sont référencés dans le Tableau 1.

Tableau 1 : Conversion de la cellulose. Rendements en acide lactique, hydroxyacétone, propylène glycol et rendement total des produits en $C_3$.

| Catalyseur | Conversion cellulose (%) | Rendement molaire (%) | | | | |
|---|---|---|---|---|---|---|
| | | Acide lactique (%) | Hydroxyacétone (HA) (%) | Propylène glycol(PG) (%) | Somme des produits HA et PG | somme des autres produits |
| Sans catalyseur | 32 | 4 | 2 | 0 | 2 | 30 |
| AlSn (C1, Exemple 1) | 44 | 23 | 3 | 0 | 3 | 41 |
| Pt/AlSn (C2, Exemple 2) | 63 | 3 | 30 | 13 | 43 | 20 |
| Pt/SiO$_2$ (C3, Exemple 3, non conforme) | 28 | 1 | 7 | 1 | 8 | 20 |

Pour le catalyseur C1, AlSn, non conforme à l'invention, la quantité d'hydroxyacétone formée représente 3% en mole de la quantité de cellulose de départ, avec 3% en mole de molécules d'hydroxyacétone et de propylène glycol La conversion est de 44%. Le rendement en propylène glycol est nul.

Pour le catalyseur C3, Pt/SiO$_2$, non conforme à l'invention, la quantité d'hydroxyacétone formée représente 7% en mole de la quantité de cellulose de départ. La quantité d'hydroxyacétone et de polypropylène glycol produite est de 8% en mole. La conversion de cellulose est de 28 %.

L'association d'une phase métallique Pt et d'un support avec une acidité de Lewis amenée par l'étain se révèle efficace en comparaison avec l'association du platine et d'un support sans acidité de Lewis (catalyseur C2 vs. catalyseur C3). On observe un rendement molaire en hydroxyacétone 4 fois plus important en présence d'étain et de platine. Le rendement en propylène glycol est également largement supérieur. La conversion de la cellulose est doublée.

De plus, l'association du platine et d'un support contenant de l'étain se montre efficace en comparaison avec un catalyseur contenant de l'étain sans platine. On observe une augmentation de la conversion totale de 19 points et de la sélectivité en molécules oxygénées en C$_3$ de 21 points dans le cas de l'alumine contenant de l'étain en présence ou non de platine. Une différence de sélectivité est observée lors de l'ajout du platine. En absence de platine, une sélectivité importante est obtenue en acide lactique. En présence de platine, une sélectivité importante en hydroxacétone et propylène glycol est obtenue.

Ainsi, ces exemples démontrent l'obtention de molécules oxygénées en C$_3$ à haut rendement et sélectivité par transformation directe de cellulose via des catalyseurs hétérogènes à base d'étain et de platine.

Exemple 5 (non-conforme à l'invention) : Transformation de la cellulose mettant en oeuvre le catalyseur C2 préparé dans l'exemple 2 à une température de 150°C.

Cet exemple concerne la conversion de la cellulose à partir du catalyseur C2 pour la production de produits C3 valorisables, et, en particulier d'hydroxyacétone et de propylène glycol.

Dans un autoclave de 100mL, on introduit 65mL d'eau, 1,6g de cellulose Avicel® (70% cristallinité) et 0,68g de catalyseur C2. L'autoclave est chauffé à 150°C et une pression de 5 MPa d'hydrogène est introduite. Après 24h de réaction, le milieu réactionnel est prélevé et centrifugé. Le liquide réactionnel est ensuite analysé par chromatographie liquide sous haute pression (HPLC) en utilisant la réfractométrie pour déterminer la teneur en produits de conversion de la solution aqueuse.

Les résultats obtenus sont référencés dans le Tableau 2.

Tableau 2 : Conversion de la cellulose. Rendements en acide lactique, hydroxyacétone, propylène glycol et rendement total des produits en C$_3$.

| Catalyseur | Conversion cellulose (%) | Rendement molaire (%) | | | | |
| | | Acide lactique (%) | Hydroxyacétone (HA) (%) | Propylène glycol (PG) (%) | Somme des produits HA et PG | somme des autres produits |
|---|---|---|---|---|---|---|
| Pt/AlSn (C2, Exemple 2) | 5 | 0.2 | 2.5 | 1 | 3.5 | 1.5 |

Le procédé selon l'invention mis en oeuvre à une température de 150 °C ne permet pas d'obtenir un rendement en hydroxyacétone et en propylène élevé. Par ailleurs, la conversion en cellulose est de 5%.

Exemple 6 (conforme à l'invention) : Transformation de la cellulose mettant en oeuvre le catalyseur C2 préparé dans l'exemple 2

Cet exemple concerne la conversion de la cellulose à partir du catalyseur C2 pour la production de produits C3 valorisables, et, en particulier d'hydroxyacétone et de propylène glycol.

Dans un autoclave de 100mL, on introduit 65mL d'eau, 1,6g de cellulose Avicel® (70% cristallinité) et 0,68g de catalyseur C2. L'autoclave est chauffé à 190°C et une pression de 8 MPa d'hydrogène est introduite. Après 24h de réaction, le milieu réactionnel est prélevé et centrifugé. Le liquide réactionnel est ensuite analysé par chromatographie liquide sous haute pression (HPLC) en utilisant la réfractométrie pour déterminer la teneur en produits de conversion de la solution aqueuse.

Les résultats obtenus sont référencés dans le Tableau 3.

Tableau 3 : Conversion de la cellulose. Rendements en acide lactique, hydroxyacétone, propylène glycol et rendement total des produits en $C_3$.

| Catalyseur | Conversion cellulose (%) | Rendement molaire (%) | | | | |
|---|---|---|---|---|---|---|
| | | Acide lactique (%) | Hydroxyacétone (HA) (%) | Propylène glycol (PG) (%) | Somme des produits HA et PG | somme des autres produits |
| Pt/AlSn (C2, Exemple 2) | 75 | 3 | 15 | 38 | 53 | 22 |

L'utilisation du catalyseur C2 selon l'invention permet l'obtention d'un rendement molaire en hydroxyacétone et en propylène glycol élevé. Le rendement en propylène glycol est également largement supérieur à ceux observé en utilisant des catalyseurs non-conformes à l'invention (C1 et C3). La conversion de la cellulose est de 75%.

Exemple 7 (conforme à l'invention): Préparation d'un catalyseur C4 à base d'oxyde d'étain dispersé sur un support à base d'oxyde d'aluminium et de ruthénium.

1g de catalyseur C1 est traité sous vide 1 h à 100°C. Une solution aqueuse de $RuC_3$ est ensuite ajoutée au catalyseur C1. Le mélange est agité pendant une heure. La solution aqueuse est évaporée. Le solide obtenu est en premier lieu séché à l'étuve à 110°C pendant 24h puis traité thermiquement sous débit d'air à la température de 300°C pendant deux heures.
Le catalyseur C4 ainsi obtenu contient 2 % poids de ruthénium et 15% poids d'étain. La proportion de sites acides de Lewis du catalyseurs C2 est supérieure à 90%.

Exemple 8 (conforme à l'invention): Préparation d'un catalyseur C5 à base d'oxyde d'étain dispersé sur un support à base d'oxyde d'aluminium et de cuivre

1 g de catalyseur C1 est traité sous vide 1 h à 100°C. Une solution aqueuse de $Cu(NO_3)_2$ est ensuite ajoutée au catalyseur C1. Le mélange est agité pendant une heure. La solution aqueuse est évaporée. Le solide obtenu est en premier lieu séché à l'étuve à 110°C pendant 24h puis traité thermiquement sous débit d'air à la température de 300°C pendant deux heures.
Le catalyseur C5 ainsi obtenu contient 10% poids de cuivre et 15% poids d'étain.
La proportion de sites acides de Lewis du catalyseurs C2 est supérieure à 90%.

Exemple 9 : Transformation de la cellulose mettant en oeuvre le catalyseur préparé à partir de catalyseurs C4 et C5

Cet exemple concerne la conversion de la cellulose à partir des catalyseurs conformes à l'invention C4 et C5 pour la production de produits C3 valorisables, et, en particulier d'hydroxyacétone et de propylène glycol.
Dans un autoclave de 100mL, on introduit 65mL d'eau, 1,6g de cellulose Avicel® (70% cristallinité) et 0,68g de catalyseur C4 ou C5. L'autoclave est chauffé à 190°C et une pression de 5 MPa d'hydrogène est introduite. Après 24h de réaction, le milieu réactionnel est prélevé et centrifugé. Le liquide réactionnel est ensuite analysé par chromatographie liquide sous haute pression (HPLC) en utilisant la réfractométrie pour déterminer la teneur en produits de conversion de la solution aqueuse.
Les résultats obtenus sont référencés dans le Tableau 4.

Tableau 4 : Conversion de la cellulose. Rendements en acide lactique, hydroxyacétone, propylène glycol et rendement total des produits en $C_3$.

| Catalyseur | Conversion cellulose (%) | Rendement molaire (%) | | | | |
|---|---|---|---|---|---|---|
| | | Acide lactique (%) | Hydroxyacétone (HA) (%) | Propylène glycol (PG) (%) | Somme des produits HA et PG | somme des autres produits |
| Ru/AlSn | 50 | 5 | 13 | 8 | 21 | 24 |
| Cu/AlSn | 37 | 2 | 12 | 9 | 21 | 16 |

L'utilisation des catalyseurs C4 et C5 selon l'invention permettent l'obtention d'un rendement molaire en hydroxyacétone et en propylène glycol élevé ainsi qu'une conversion en cellulose élevée.

**Revendications**

1. Procédé de transformation de biomasse lignocellulosique ou de cellulose en hydroxyacétone et en propylène glycol, dans lequel la biomasse lignocellulosique ou la cellulose est mise en contact, en conditions hydrothermales, sous atmosphère réductrice, avec un catalyseur hétérogène à base d'oxyde d'étain et/ou d'antimoine et contenant au moins un élément à l'état métallique choisi parmi Pt, Ni, Ru, Cu, ledit catalyseur présentant des sites acides de type Lewis, ladite teneur en sites acides de Lewis étant supérieure à 50% de la teneur en sites acides totale, ledit catalyseur est dispersé sur un support à base d'oxydes choisi parmi les oxydes d'aluminium, et/ou de zirconium, et/ou de titane, et/ou de niobium et/ou de silicium, ledit procédé étant effectué à une température comprise entre 160 et 250°C, et à une pression comprise entre 0,5 et 20 MPa.

2. Procédé selon la revendication 1 dans lequel la teneur pondérale de l'élément à l'état métallique est comprise entre 0,01 et 10% poids par rapport à la masse totale du catalyseur.

3. Procédé selon l'une des revendications précédentes dans lequel la teneur en étain et/ou antimoine est comprise entre 1 et 100% poids, par rapport à la masse totale du catalyseur.

4. Procédé selon l'une des revendications précédentes dans lequel le catalyseur est synthétisé par échange ionique ou par imprégnation, suivi d'un traitement thermique.

5. Procédé selon l'une des revendications précédentes dans lequel la transformation est mise en oeuvre dans un milieu contenant de l'eau, ledit milieu étant choisi parmi le groupe formé par un milieu liquide, un liquide ionique et un milieu supercritique de densité type liquide.

6. Procédé selon la revendication précédente dans lequel la teneur massique en eau est supérieure à 1%.

7. Procédé selon l'une des revendications précédentes dans lequel l'atmosphère réductrice est une atmosphère d'hydrogène, pure ou en mélange.

8. Procédé selon l'une des revendications précédentes dans lequel la transformation est effectuée à une température comprise entre 175 et 250°C, et à une pression comprise entre 2 et 10 MPa.

9. Procédé selon l'une des revendications précédentes dans lequel le catalyseur est introduit avec un rapport massique biomasse/catalyseur compris entre 1 et 1000.

10. Procédé selon l'une des revendications précédentes dans lequel le catalyseur subit une étape de traitement thermique réducteur à une température comprise entre 200 et 600°C sous flux ou atmosphère d'hydrogène préalablement à l'introduction de la biomasse lignocellulosique dans le réacteur.

11. Procédé selon l'une des revendications précédentes dans lequel la biomasse lignocellulosique ou la cellulose est introduite avec un rapport massique (milieu contenant de l'eau)/biomasse compris entre 1 et 1000.

12. Procédé selon l'une des revendications précédentes **caractérisé en ce qu'**il est mis en oeuvre en discontinu ou en continu.

13. Procédé selon la revendication 12 **caractérisé en ce qu'**il est mis en oeuvre en continu avec une vitesse massique horaire comprise entre 0,01 et 5 $h^{-1}$.

**Patentansprüche**

1. Verfahren zur Umwandlung von lignocellulosehaltiger Biomasse oder von Cellulose in Hydroxyaceton und in Propylenglykol, bei dem die lignocellulosehaltige Biomasse oder die Cellulose unter hydrothermalen Bedingungen in eine reduzierenden Atmosphäre mit einem heterogenen Katalysator auf Basis von Zinnoxid und/oder Antimonoxid in Kontakt gebracht werden, der wenigstens ein Element im metallischen Zustand ausgewählt aus Pt, Ni, Ru, Cu enthält, wobei der Katalysator saure Stellen vom Lewis-Typ aufweist, wobei der Gehalt an Lewis-sauren Stellen mehr als 50% der gesamten sauren Stellen beträgt, wobei der Katalysator auf einer Basis von Oxiden dispergiert wird, ausgewählt aus den Oxiden von Aluminium, und/oder von Zirkonium, und/oder von Titan, und/oder von Niob, und/oder Silicium, wobei das Verfahren bei einer Temperatur zwischen 160 und 250°C und bei einem Druck zwischen 0,5 und 20 MPa durchgeführt wird.

2. Verfahren gemäß Anspruch 1, bei dem der Gehalt des Elements im metallischen Zustand zwischen 0,01 und 10 Gew.-% im Verhältnis zur Gesamtmasse des Katalysators beträgt.

3. Verfahren gemäß einem der vorherigen Ansprüche, bei dem der Gehalt an Zinn und/oder Antimon zwischen 1 und 100 Gew.-% im Verhältnis zur Gesamtmasse des Katalysators beträgt.

4. Verfahren gemäß einem der vorherigen Ansprüche, bei dem der Katalysator hergestellt wird durch Ionenaustausch oder durch Imprägnation, gefolgt von einer thermischen Behandlung.

5. Verfahren gemäß einem der vorherigen Ansprüche, bei dem die Umwandlung in einem wasserhaltigen Medium durchgeführt wird, wobei das Medium ausgewählt ist aus der Gruppe gebildet durch ein flüssiges Medium, ein ionisches Medium und einem überkritischen Medium mit der Dichte vom Flüssigkeitstyp.

6. Verfahren gemäß einem der vorherigen Ansprüche, bei dem der Massengehalt von Wasser größer als 1% ist.

7. Verfahren gemäß einem der vorherigen Ansprüche, bei dem die reduzierende Atmosphäre eine reine oder gemischte Wasserstoffatmosphäre ist.

8. Verfahren gemäß einem der vorherigen Ansprüche, bei dem die Umwandlung bei einer Temperatur zwischen 175 und 250°C und bei einem Druck zwischen 2 und 10 MPa durchgeführt wird.

9. Verfahren gemäß einem der vorherigen Ansprüche, bei dem der Katalysator eingeführt wird mit einem Masseverhältnis von Biomasse/Katalysator enthalten zwischen 1 und 1000.

10. Verfahren gemäß einem der vorherigen Ansprüche, bei dem der Katalysator eine Stufe der thermischen Reduktion bei einer Temperatur zwischen 200 und 600°C erfährt, unter einem Strom oder einer Atmosphäre aus Wasserstoff, vor Einführung der lignosecellulosehaltigen Biomasse in den Reaktor.

11. Verfahren gemäß einem der vorherigen Ansprüche, bei dem die lignosecellulosehaltige Biomasse oder die Cellulose mit einem Masseverhältnis (Medium enthaltend Wasser)/Biomasse eingeführt wird zwischen 1 und 1000.

12. Verfahren gemäß einem der vorherigen Ansprüche, **dadurch gekennzeichnet, dass** es diskontinuierlich oder kontinuierlich durchgeführt wird.

13. Verfahren gemäß Anspruch 12, **dadurch gekennzeichnet, dass** es kontinuierlich durchgeführt mit einer stündlichen Massengeschwindigkeit zwischen 0,01 und $5h^{-1}$.

## Claims

1. Process for transformation of lignocellulosic biomass or cellulose into hydroxyacetone and propylene glycol, in which the lignocellulosic biomass or cellulose is brought into contact, under hydrothermal conditions and under a reducing atmosphere, with a heterogeneous catalyst that is based on tin oxide and/or antimony oxide and that contains at least one element in the metal state that is selected from among Pt, Ni, Ru, Cu, with said catalyst having Lewis-type acid sites, said content of Lewis acid sites being greater than 50% of the total acid site content, said catalyst being dispersed on a substrate based on oxides selected from among the group that is formed by the oxides of aluminum and/or zirconium and/or titanium and/or niobium and/or silicon, said process being carried out at a temperature of between 160 and 250°C, and at a pressure of between 0.5 and 20 MPa.

2. Process according to claim 1, in which the content by weight of the element in the metal state is between 0.01 and 10% by weight relative to the total mass of the catalyst.

3. Process according to one of the preceding claims, in which the content of tin and/or antimony is between 1 and 100% by weight, relative to the total mass of the catalyst.

4. Process according to one of the preceding claims, in which the catalyst is synthesized by ion exchange or by impregnation, followed by a heat treatment.

5. Process according to one of the preceding claims, in which the transformation is mplemented in a water-containing medium, with said medium being selected from among the group that is formed by a liquid medium, an ionic liquid, and a supercritical medium of liquid-type density.

6. Process according to the preceding claim, in which the content by mass of water is greater than 1%.

7. Process according to one of the preceding claims, in which the reducing atmosphere is a hydrogen atmosphere, in pure form or in a mixture.

8. Process according to one of the preceding claims, in which the transformation is carried out at a temperature of between 175 and 250°C, and at a pressure of between 2 and 10 MPa.

9. Process according to one of the preceding claims, in which the catalyst is introduced with a biomass/catalyst mass ratio of between 1 and 1,000.

10. Process according to one of the preceding claims, in which the catalyst undergoes a reducing heat treatment stage at a temperature of between 200 and 600°C under a stream or atmosphere of hydrogen prior to the introduction of the lignocellulosic biomass into the reactor.

11. Process according to one of the preceding claims, in which the lignocellulosic biomass or cellulose is introduced with a (water-containing medium)/biomass mass ratio of between 1 and 1,000.

12. Process according to one of the preceding claims, **characterized in that** it is implemented intermittently or continuously.

13. Process according to Claim 15, wherein it is implemented continuously with a mass speed per hour of between 0.01 and 5 $h^{-1}$.

## RÉFÉRENCES CITÉES DANS LA DESCRIPTION

**Documents brevets cités dans la description**

- EP B2011569 A1
- WO 03035582 A
- US 2488722 A

**Littérature non-brevet citée dans la description**

- **LUO et al.** *Angew. Chem. Int. Ed.,* 2007, vol. 46, 7636-7639
- **JI et al.** *Angew. Chem. Int. Ed.,* 2008, vol. 47, 8510-8513
- **ZHANG et al.** *Chem. Commun.,* 2010, vol. 46, 862-864
- *Science china chemistry,* 2010, vol. 53, 1476-4780
- **PATWARDHAN et al.** *Bioresource Technology,* 2010, vol. 101, 4646-4655
- **M. GUISNET ; P. AYRAULT ; C. COUTANCEAU, ; M.F. ALVAREZ ; J. DATKA.** *J. Chem. Soc., Faraday Trans.,* 1997, vol. 93, 1661